# EUROPEAN PATENT APPLICATION

(11) **EP 4 620 988 A1**
(43) Date of publication of application: **24.09.2025**
(21) Application number: 23907007.1
(22) Date of filing: 19.12.2023
(51) Int. Cl.: C08F 220/34, A61K 31/785, A61K 31/80, A61K 47/36, A61K 47/42, A61P 27/02, C08F 220/60, C08F 230/02, C08L 33/26

(54) **COPOLYMER, AND COMPOSITION FOR EYES**

(30) Priority: 21.12.2022 JP 2022204230
(71) Applicant: NOF Corporation, Shibuya-ku Tokyo 150-6019 (JP)
(72) Inventor: SASAKI, Ayano, Kawasaki-shi, Kanagawa 210-0865 (JP); KAWASAKI, Hiroko, Kawasaki-shi, Kanagawa 210-0865 (JP); IWAKIRI, Norio, Kawasaki-shi, Kanagawa 210-0865 (JP)
(74) Representative: Patentanwaltskanzlei WILHELM & BECK
(86) International application number: PCT/JP2023/045409
(87) International publication number: WO 2024/135647

(57) **Abstract**

A copolymer containing a structural unit derived from a monomer (a) of formula (1a) or a salt thereof and from a monomer (b) of formula (1b) or (2b), having an Mw of 10,000-2,000,000, the molar ratio nₐ:n_{b} being 10:90 to 70:309. 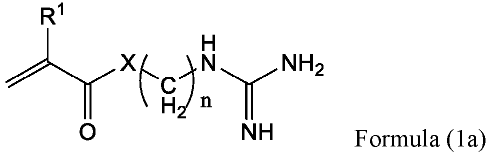 (R¹ is H or CH₃, X is O or NR², R² is H or a C1-4 alkyl group, and n=1-4.) 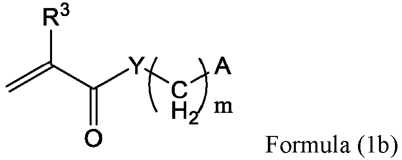 (R³ is H or CH₃, Y is O or NR⁴, R⁴ is H or a C1-4 alkyl group, m is 1-6, there may be a hydroxyl group in a carbon chain, and A is a phosphorylcholine group or a hydroxyl group, and may be H when m=1 or when there is a hydroxyl group in a carbon chain.) 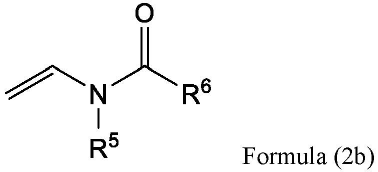 (R⁵ is H or CH₃, R⁶ is a C1-4 alkyl group or an alkylene group that forms a cycling structure together with R⁵.)

## Description

### Technical Field

The present invention relates to a copolymer and an ophthalmic composition using the same.

### Background Art

Recently, the number of potential dry eye patients in Japan has been said to be as high as 8 million from a decrease in the number of blinks due to the use of digital devices for a long period of time and due to an increase in contact lens wearers. It is known that dry eye is induced by decreased tear secretion from the lacrimal gland and evaporation of water due to abnormalities in the lipids and mucin in tears, leading to decreased function of the cornea and conjunctiva, inflammatory diseases, and the like.

To prevent and treat the dry eyes that cause these ocular diseases, eye drops having water-retaining properties to prevent insufficient moisture are known. For example, PTL 1 teaches that eye drops having hyaluronic acid or a salt thereof and a cellulose-based polymer or a vinyl-based polymer can improve the dry eye symptoms.

On the other hand, the number of contact lens users is said to be as high as 15 million in Japan, and because of its convenience, the wearing time of contact lens has prolonged. It has been reported that the average daily wearing time in young contact lens wearers is about 14 hours, but wearing contact lenses for a long period of time can easily lead to discomfort such as dryness and a gritty feeling. Thus, there remains a need from the market for contact lenses with a sustained good wearing feel.

Discomfort when wearing contact lenses has been attributed to dryness and friction on the surface of contact lenses. As a method of solving these, for example, a method of providing moisture retention and lubricity to a contact lens as described in PTLs 2 and 3 is known. PTL 2 discloses a method of blending a hydrophilic polymer such as hyaluronic acid into a contact lens packing liquid. In addition, PTL 3 discloses a liquid for attaching contact lenses which uses a polyvinyl polymer compound, a cellulose polymer compound, or the like.

### Citation List

### Patent Literature

PTL 1
   Japanese Patent Application Laid-Open No. 2014-167034
PTL 2
   WO2013/031020
PTL 3
   Japanese Patent Application Laid-Open No. 2005-187345

### Summary of Invention

### Technical Problem

Although the above-mentioned cellulosic polymer and vinyl-based polymer described in PTL 1 can alleviate dry eye symptoms by improving water retention, neither of them has sufficient retention on the cornea, and there is a problem in sustainability of effect. In addition, the methods described in PTLs 2 and 3 cannot achieve sufficient retention on the contact lens, and the components tend to wash off over time. Therefore, there is a demand for a compound that can remain on the corneal surface and the contact lens surface for a long period of time and can retain water retention and lubricity. Here, according to the findings of the present inventors, it is considered that a compound which interacts with mucin present on the surface of corneal epidermal cells or mucin secreted in tears can remain on the corneal surface and the contact lens surface for a long period of time.

In view of the above problems, it is an object of the present invention to provide a copolymer which interacts with mucin and can sustain water retention and lubricity for a long period of time, and an ophthalmic composition using the same.

### Solution to Problem

As a result of extensive studies, the present inventors have found that a novel copolymer comprising a monomer having a guanidino group and a particular hydrophilic monomer can achieve the above object, and thus has completed the present invention.

That is, the present invention consists of the following [1] to [7].
[1] A copolymer containing: a constituent unit derived from a monomer (a) represented by a formula (1a) below or a salt thereof; and a constituent unit derived from a monomer (b) represented by any one of formulas (1b) and (2b) below:
   in which
   a molar ratio nₐ:n_{b} between the constituent units is 10:90 to 70:30, nₐ being a molar proportion of the constituent unit derived from the monomer (a) or the salt thereof in the copolymer, n_{b} being a molar proportion of the constituent unit derived from the monomer (b) in the copolymer, and
   a weight average molecular weight of the copolymer is 10,000 to 2,000,000. 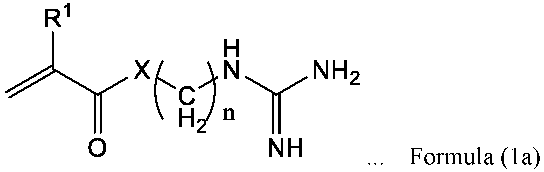
   (In formula (1a), R¹ is a hydrogen atom or a methyl group, X is O or NR² where R² is H or an alkyl group having 1 to 4 carbon atoms, and n is 1 to 4.) 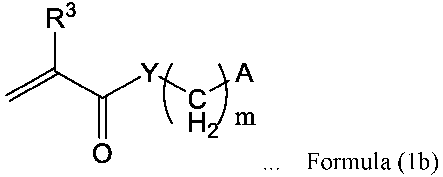
   (In formula (1b), R³ is a hydrogen atom or a methyl group, Y is O or NR⁴ where R⁴ is H or an alkyl group having 1 to 4 carbon atoms, m is 1 to 6, a carbon chain of the formula (1b) optionally has at least one hydroxyl group, and A is a phosphorylcholine group or a hydroxyl group and optionally is a hydrogen atom only in a case where m is 1 or in a case where the carbon chain has the at least one hydroxyl group.) 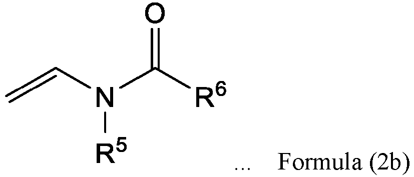
   (In formula (2b), R⁵ is a hydrogen atom or a methyl group, and R⁶ is an alkyl group having 1 to 4 carbon atoms or an alkylene group having 1 to 4 carbon atoms whose terminal is bonded to R⁵ to form a cyclic structure.)
[2] The copolymer according to [1], in which
   the monomer (b) contains at least one monomer selected from the group consisting of monomers represented by a formula (3b) below, N-vinylpyrrolidone, hydroxyethyl (meth)acrylate, hydroxypropyl (meth)acrylate, hydroxybutyl (meth)acrylate, hydroxyethyl (meth)acrylamide, glycerol mono(meth)acrylate, N,N-dimethyl (meth)acrylamide, N-vinyl acetamide, and N-vinyl-N-methyl acetamide. 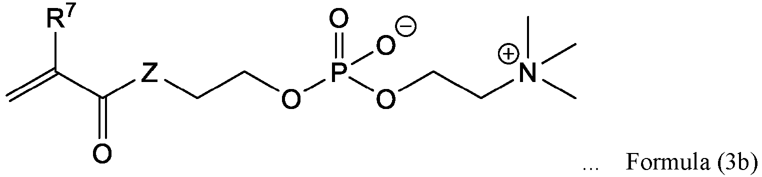
   (In formula (3b), R⁷ is a hydrogen atom or a methyl group, and Z is O or NR⁸ where R⁸ is H or an alkyl group having 1 to 4 carbon atoms.)
[3] The copolymer according to [1] or [2], in which the monomer (b) contains at least one of a monomer represented by the formula (3b) and N-vinylpyrrolidone.
[4] The copolymer according to any one of [1] to [3], in which the monomer (b) contains 2-(methacryloyloxy)ethyl 2-(trimethylammonio)ethyl phosphate.
[5] The copolymer of any one of [1] to [4], in which the monomer (a) or the salt thereof contains a monomer represented by a formula (2a) below or a salt thereof. 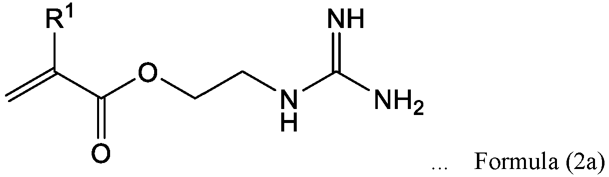 (In formula (2a), R¹ is a hydrogen atom or a methyl group.)
[6] An ophthalmic composition containing: 0.001 w/v% or more and 5.0 w/v% or less of the copolymer (P) according to any one of [1] to [5].
[7] The ophthalmic composition according to [6], further containing: 0.001 w/v % or more and 10.0 w/v % or less of a polysaccharide or a glycoprotein.

### Advantageous Effects of Invention

A copolymer of the present invention provides water retention and lubricity to the corneal surface or the contact lens surface, and can remain on the corneal surface or the contact lens surface for a long period of time to provide these benefits in a sustained manner. For this reason, copolymer of the present invention is useful as an ophthalmic composition and is suitable as eye drops or a contact lens treatment liquid.

### Brief Description of Drawings

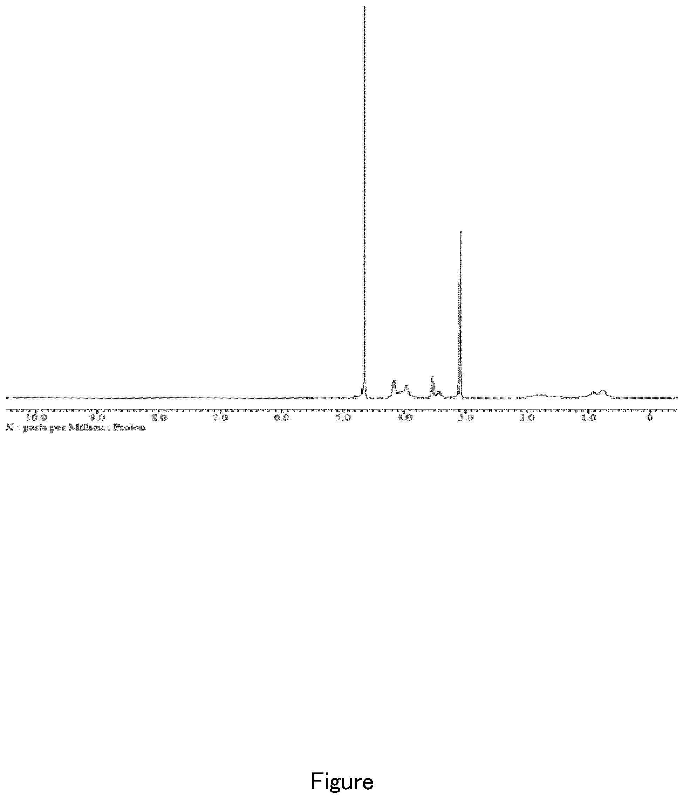

The figure illustrates the results of ¹H NMR measurement of a copolymer (P1) of the Example.

### Description of Embodiments

Hereinafter, the present invention will be described in more detail.

In the present specification, when a preferable numerical range (for example, a range of concentration or weight average molecular weight) is described stepwise, each lower limit and upper limit can be combined independently. For example, in the description "preferably 10 to 100, more preferably 20 to 90," a combination of "preferable lower limit value of 10" and "more preferable upper limit value of 90" can be combined to "10 or more and 90 or less." In other words, the range can be 10 to 90.

In the present invention, "(meth)acrylic" means acrylic or methacrylic, "(meth)acryloyl" means acryloyl or methacryloyl, "(meth)acrylate" means acrylate or methacrylate, and "(meth) acrylamide" means acrylamide or methacrylamide

Examples of the product form of an ophthalmic composition of the present invention include compositions, such as a composition to be applied directly to an eye, and a composition to be used for processing an apparatus to be attached to an eye, such as a contact lens, and can be specified as follows. Specific examples of the product form include eye drops, eyewash, and contact lens treatment liquid. Specific examples of contact lens treatment liquid include contact lens packing liquids (contact lens shipping liquids), contact lens preservation liquids, contact lens cleaning liquids, contact lens cleaning and preservation liquids, contact lens disinfectants, agents for attaching contact lens, and the like.

Herein, a contact lens packing liquid is an aqueous solution which is enclosed in a packaging container such as a blister package together with a contact lens for distributing contact lenses. In general, contact lenses are used in a state of being swollen with an aqueous solution (contact lens packing liquid). Therefore, at the time of shipment, the lenses are enclosed in packaging containers in this swollen state so that they can be used immediately.

### [Copolymer (P)]

A copolymer (P) of the present invention is obtained by polymerizing a monomer (a) represented by the below formula (1a) or a salt thereof and a monomer (b) represented by any one of the below formulas (1b) and (2b). Therefore, the copolymer (P) includes a constituent unit derived from the monomer (a) or a salt thereof and a constituent unit derived from the monomer (b). The copolymer (P) has a molar ratio nₐ: n_{b} of 10:90 to 70:30 between the constituent units derived from the respective constituent monomers, and a weight average molecular weight of 10,000 to 2,000,000.

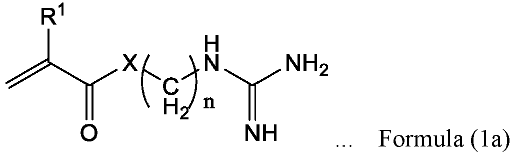

In the formula (1a), R¹ is a hydrogen atom or a methyl group, and X is O or NR² where R² is H or an alkyl group having 1 to 4 carbon atoms, and n is 1 to 4.

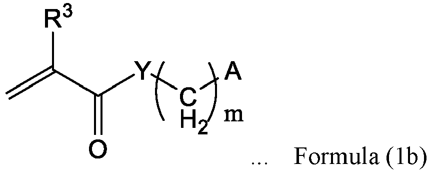

In the formula (1b), R³ is a hydrogen atom or a methyl group, Y is O or NR⁴ where R⁴ is H or an alkyl group having 1 to 4 carbon atoms, m is 1 to 6, the carbon chain of the formula (1b) may have at least one hydroxyl group, and A is a phosphorylcholine group or a hydroxyl group and may be a hydrogen atom only in the case where m is 1 or in the case where the carbon chain has a hydroxyl group.

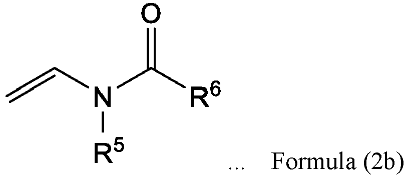

In the formula (2b), R⁵ is a hydrogen atom or a methyl group, and R⁶ is an alkyl group having 1 to 4 carbon atoms or an alkylene group having 1 to 4 carbon atoms whose terminal is bonded to R⁵ to form a cyclic structure.

### [Monomer (a)]

The copolymer (P) of the present invention is obtained by using a monomer (a) having a guanidino group represented by the formula (1a) (hereinafter, also referred to as "GE monomer (a)") or a salt thereof in polymerization. The types of salts include hydrochlorides, carbonates, bicarbonates, phosphates, sulfates, and the like. The salt is preferably a hydrochloride, a carbonate or a bicarbonate, and more preferably a hydrochloride. When a copolymer (P) has a GE monomer (a) or a salt thereof as the constituent monomer, it is possible to facilitate the interaction of the copolymer (P) with mucin and to enhance the retention on the corneal surface and the contact lens surface. The GE monomer (a) is not limited to one type, and one or more types thereof may be used.

The GE monomer (a) is preferably a monomer of formula (1) where n is 2.

A preferred examples of the GE monomer (a) where n is 2 is a monomer represented by the formula (2a) where X is O.

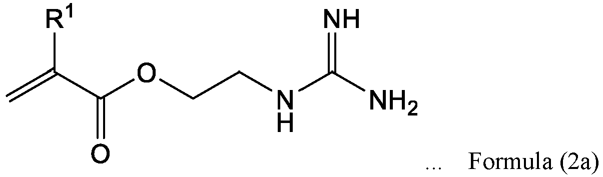

In the formula (2a), R¹ is a hydrogen atom or a methyl group.

A more preferred example of the monomer represented by the formula (2a) is 2-guanidinoethyl methacrylate where R¹ is a methyl group.

Another preferred example of the GE monomer (a) where n is 2 is 2-guanidinoethyl methacrylamide where R¹ is a methyl group, X is NR³, and R³ is H.

The monomer represented by the formula (1a) can be produced by a known method, for example, a method in which amino group-containing polymerizable monomer and 1-amidinopyrazole hydrochloride are reacted in the presence of a base, as described in Barner-Kowollik, Christopher, et al, Polymer Chemistry (2020), 11 (26), 4213-4220 (hereinafter, Reference 1) and Zhao, Qingqing, et al, Chemical Communications (2020), 56 (36), 4954-4957 (hereinafter, Reference 2) and the like.

### [Monomer (b)]

The copolymer (P) of the present invention is obtained by using a monomer (b) represented by any one of the formulas (1b) and (2b) for polymerizing. By using a hydrophilic monomer (b) for the copolymerization, the drying resistance of ophthalmic composition containing the copolymer (P) can be increased on the cornea or contact lens surface.

Preferable examples of the monomer (b) include hydroxyalkyl (meth)acrylate in which Y is O and A is a hydroxyl group in the formula (1b); hydroxyalkyl (meth)acrylamide in which Y is NH and A is a hydroxyl group; N,N-dimethyl(meth)acrylamide in which Y is NR⁴, R⁴ is a methyl group, m is 1, and A is a hydrogen atom; glycerol mono(meth)acrylate in which Y is O, m is 3, and a hydroxyl group is at the 2-position, and A is a hydroxyl group; and a monomer represented by the formula (3b) in which m is 2 and A is a phosphorylcholine group. Other preferable example of the monomer (b) include N-vinylacetamide or N-methyl N-vinylacetamide in which R6 in the formula (2b) is a methyl group; N-vinylpyrrolidone in which R6 is an ethylene group, and has a cyclic structure; and the like. More preferred examples include monomers represented by the formula (3b) below, N-vinylpyrrolidone, hydroxyethyl (meth)acrylate, hydroxypropyl (meth)acrylate, hydroxybutyl (meth)acrylate, hydroxyethyl (meth)acrylamide, glycerol mono(meth)acrylate, N,N-dimethyl (meth)acrylamide, N-vinyl acetamide, N-vinyl-N-methyl acetamide, and N-vinylpyrrolidone. Further preferred monomer (b) is a monomer represented by the formula (3b) or N-vinylpyrrolidone, and still more preferably a monomer represented by the formula (3b). When a monomer represented by the formula (3b) or N-vinylpyrrolidone, particularly a monomer represented by the formula (3b), is used, it is possible to further enhance the drying resistance of an ophthalmic composition containing the copolymer (P) on a cornea or a contact lens surface. The monomer (b) is not limited to one type, and one or more types thereof may be used.

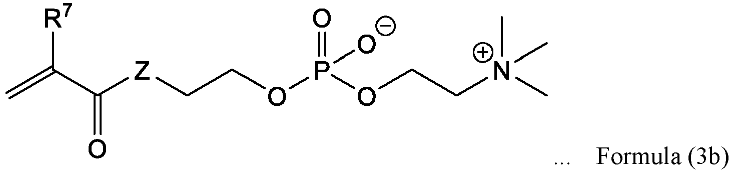

In the formula (3b), R⁷ is a hydrogen atom or a methyl group and Z is O or NR⁸ where R⁸ is H or an alkyl group having 1 to 4 carbon atoms.

Examples of the monomer represented by the formula (3b) include 2-((meth)acryloyloxy)ethyl-2-(trimethylammonio)ethyl phosphate and 2-((meth)acrylamido)ethyl-2-(trimethylammonio)ethyl phosphate, and more preferably, 2-(methacryloyloxy)ethyl 2-(trimethylammonio)ethyl phosphate (also known as 2-methacryloyloxyethyl phosphorylcholine).

A monomer represented by the formula (3b) can be produced by a known method, and examples thereof include a method in which a hydroxyl group-containing polymerizable monomer is reacted with 2-bromoethylphosphoryl dichloride in the presence of a tertiary base, and the compound thus obtained is reacted with a tertiary amine. In addition, the following method may be used: a cyclic compound is obtained by reacting a hydroxyl group-containing polymerizable monomer with a cyclic phosphorus compound, and then a ring-opening reaction is performed with a tertiary amine.

### [Ratio between GE monomer (a) and monomer (b)]

The molar ratio nₐ:n_{b} in the copolymer (P) between constituent unit derived from the GE monomer (a) or a salt thereof and constituent unit derived from the monomer (b) is 10:90 to 70:30, preferably 20:80 to 60:40, and even more preferably 30:70 to 50:50. Here, nₐ is the molar proportion of the constituent unit derived from the GE monomer (a) or a salt thereof in the copolymer (P), and n_{b} is the molar proportion of the constituent unit derived from the monomer (b) in the copolymer (P). In addition, a plurality of types of GE monomers (a) may be used in combination at any ratio. In this case, nₐ refers to the number of moles of all GE monomers combined. Similarly, a plurality of types of monomers (b) may be used in combination at any ratio. In this case, n_{b} refers to the number of moles of all monomers (b) combined.

By increasing nₐ or decreasing n_{b}, the interaction of the copolymer (P) with the ocular surface or contact lens surface can be enhanced, allowing the copolymer (P) to remain on the corneal surface or the contact lens surface for a longer period of time, and the lubricity imparted to the cornea or contact lens by an ophthalmic composition containing the copolymer (P) can also be increased. It is also possible to increase the solubility of the copolymer (P) in water and facilitate the preparation of ophthalmic composition by increasing n_{b}.

### [Other monomers]

The copolymer (P) of the present invention may contain a monomer (c) in addition to the GE monomer (a) and the monomer (b) as long as the effect of the present invention is not impaired.

The monomer (c) can be arbitrarily selected from monomers (c) copolymerizable with a GE monomers (a) and/or a monomer (b).

Examples of the monomers (c) include styrene sulfonic acid, (meth)acryloyloxyphosphonic acid, 2-hydroxy-3-(meth)acryloyloxypropyltrimethylammonium chloride, polyethylene glycol (meth)acrylate, aminoethyl (meth)acrylate, N,N-dimethylaminoethyl (meth)acrylate, N-acryloylmorpholine, ethyl (meth)acrylate, propyl (meth)acrylate, butyl (meth)acrylate, cyclohexyl (meth)acrylate, 2-ethylhexyl (meth)acrylate, n-octyl (meth)acrylate, isononyl (meth)acrylate, dodecyl (meth)acrylate, stearyl (meth)acrylate, styrene, α-methylstyrene, vinyltoluene, indene, vinylnaphthalene, vinylaniline, alkenes such as ethylene, propylene, butadiene, isobutylene, and octene, acrylonitrile, methacrylonitrile, polypropylene glycol (meth)acrylate, and glycidyl (meth)acrylate, and one or more types thereof may be used. The ratio of the monomer (c) to all the monomers used in preparing the copolymer of the present invention is preferably set to be 30 mol% or less, more preferably 15 mol% or less. The lower limit of the ratio of the monomer (c) is not particularly limited, but may be set to 0 mol% or more. This is because the effect of the present invention is satisfactorily manifested.

### [Weight average molecular weight of copolymer (p)]

The weight average molecular weight of the copolymer (P) used in the present invention is 10,000 to 2,000,000, preferably 100,000 to 1,000,000, more preferably 150,000 to 600,000, and still more preferably 200,000 to 400,000. By increasing the weight average molecular weight of the copolymer (P) to greater than 10,000, it is possible for the copolymer (P) to remain on the corneal surface or on the surface of the contact lens for a longer period of time. In addition, the ophthalmic composition containing the copolymer (P) can enhance the lubricity and water retention properties imparted to a cornea or a contact lens. As a consequence, the drying resistance of ophthalmic composition containing the copolymer (P) on corneal or contact lens surfaces can be enhanced. Reducing the weight average molecular weight of the copolymer (P) to less than 2,000,000 can facilitate the handling of the copolymer (P).

The weight average molecular weight of the copolymer (P) is determined in terms of pullulan by GPC (gel filtration chromatography) measurement.

### [Polymerized form of copolymer (P)]

A copolymer (P) is usually a random copolymer, but may be an alternating copolymer or a blocked copolymer in which the monomers are regularly arranged, and may have a graft structure in a part thereof.

### [Method for producing copolymer (P)]

The copolymer (P) can be produced by copolymerizing a GE monomer (a) having a guanidino group represented by the following formula (1a) and a monomer (b) represented by any one of the following formulas (1b) and (2b).

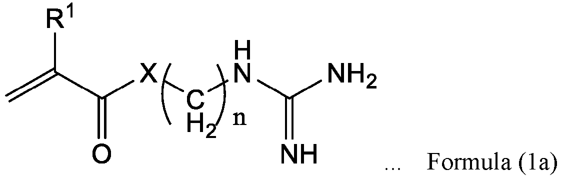

In the formula (1a), R¹ is a hydrogen atom or a methyl group, and X is O or NR² where R² is H or an alkyl group having 1 to 4 carbon atoms, and n is 1 to 4.

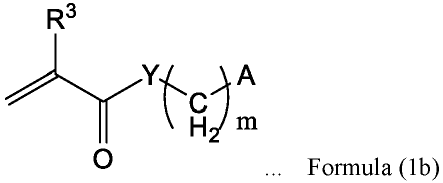

In the formula (1b), R³ is a hydrogen atom or a methyl group, Y is O or NR⁴ where R⁴ is H or an alkyl group having 1 to 4 carbon atoms, m is 1 to 6, the carbon chain of the formula (1b) may have at least one hydroxyl group, A is a phosphorylcholine group or a hydroxyl group and may be a hydrogen atom only in the case where m is 1 or in the case where the carbon chain has a hydroxyl group.

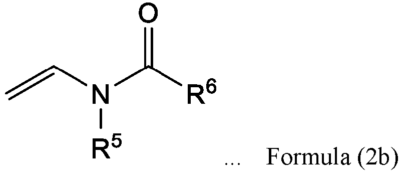

In the formula (2b), R⁵ is a hydrogen atom or a methyl group, and R⁶ is an alkyl group having 1 to 4 carbon atoms or an alkylene group having 1 to 4 carbon atoms whose terminal is bonded to R⁵ to form a cyclic structure.

The above polymerization reaction can be performed in the presence of a radical polymerization initiator in an atmosphere purged with an inert gas such as nitrogen, carbon dioxide, argon, or helium by a known method such as radical polymerization, for example, bulk polymerization, suspension polymerization, emulsion polymerization, or solution polymerization. From the viewpoint of purification, solution polymerization is preferred. Purification of copolymer can be performed by a general purification method such as a reprecipitation method, a dialysis method, or an ultrafiltration method.

Examples of the radical polymerization initiator include azo-based radical polymerization initiators, organic peroxides, and persulfates.

Examples of the azo-based radical polymerization initiators include 2,2'-azobis(2-methylpropionamidine) dihydrochloride (V-50), 2,2'-azobis(2-diaminopropyl) dihydrochloride, 2,2'-azobis(2-(5-methyl-2-imidazolin-2-yl)propane) dihydrochloride, 4,4'-azobis(4-cyanovaleric acid), 2,2-azobisisobutyramide dihydrate, 2,2-azobis(2,4-dimethylvaleronitrile), and 2,2-azobisisobutyronitrile (AIBN).

Examples of the organic peroxides include t-butyl peroxy neodecanoate (Perbutyl (registered trademark) ND), benzoyl peroxide, diisopropyl peroxydicarbonate, t-butyl peroxy-2-ethylhexanoate, t-butyl peroxy pivalate, t-butyl peroxy diisobutyrate, lauroyl peroxide, and succinic acid peroxide (succinyl peroxide).

Examples of the persulfates include ammonium persulfate, potassium persulfate, and sodium persulfate.

As the radical polymerization initiator, an azo-based radical polymerization initiator is preferably used, and 2,2'-azobis(2-methylpropionamidine) dihydrochloride (V-50) is more preferably used.

These radical polymerization initiators can be used alone or in combination of two or more types thereof. The amount of the polymerization initiator to be used is usually from 0.001 to 10 parts by mass, preferably from 0.01 to 5.0 parts by mass, based on 100 parts by mass of the monomer composition of copolymer (P).

The polymerization reaction can be performed in the presence of a solvent, and as the solvent, a solvent that allows the monomer composition to dissolves therein and does not react with the monomer composition can be used. Examples of the solvent include water; alcohol-based solvents such as methanol, ethanol, n-propanol, and isopropanol; ketone-based solvents such as acetone, methyl ethyl ketone, and diethyl ketone; ester-based solvents such as ethyl acetate; linear or cyclic ether-based solvents such as ethyl cellosolve, tetrahydrofuran, and N-methylpyrrolidone; and nitrogen-containing solvents such as acetonitrile and nitromethane. Preferably, water, alcohol or a mixed solvent thereof is used, and more preferably, water.

### [Ophthalmic composition of the present invention]

In the ophthalmic composition of the present invention, the concentration of the copolymer (P) is 0.001 w/v% or more, preferably 0.01 w/v% or more, and more preferably 0.1 w/v% or more. Further, the concentration of the copolymer (P) is 5.0 w/v% or less, preferably 2.0 w/v% or less, and more preferably 0.7 w/v% or less. By setting the concentration of the copolymer (P) to 0.001 w/v% or more, sufficient lubricity and water retention can be obtained, and from the viewpoint of manufacturability of the ophthalmic composition, it is desirable to set the concentration of the copolymer (P) to 5.0 w/v% or less.

In addition, "w/v%" in the present invention represents the mass of a certain component in grams (g) per 100 mL of solution. For example, "a composition of the present invention contains 1.0 w/v% of the copolymer (P)" means that a solution of 100 mL contains the copolymer (P) of 1.0g.

As the solvent used in an ophthalmic composition of the present invention, water, an alcohol such as ethanol, n-propanol, isopropanol, glycerol, or propylene glycol, or a mixed solvent thereof can be used. Preferably, the solvent is water or a mixed solvent of water and alcohol, and more preferably water.

Water used in ophthalmic composition of the present invention is usually water used for manufacturing a pharmaceutical or a medical device. Specifically, ion-exchanged water, purified water, sterile purified water, distilled water, and water for injection can be used.

The ophthalmic composition of the present invention may include a buffering agent. When an ophthalmic composition contains a buffering agent, pH and the osmotic pressure can be adjusted, and the feeling upon use can be further improved.

In the present invention, from the viewpoint of more sufficiently obtaining the above effect, it is desirable to use one or more types of buffering agent selected from phosphate buffers and borate buffers. Herein, a phosphate buffer is a buffer composed of disodium hydrogen phosphate, sodium dihydrogen phosphate, anhydrous sodium dihydrogen phosphate, potassium dihydrogen phosphate, dipotassium hydrogen phosphate, hydrochloric acid, sodium hydroxide, potassium hydroxide, and the like. A borate buffer is a buffer composed of boric acid, borax, hydrochloric acid, sodium hydroxide, potassium hydroxide, and the like.

The concentration of the buffering agent in the ophthalmic composition of the present invention is preferably 0.0001 w/v% or more and 15 w/v% or less, more preferably 0.001 w/v% or more and 10 w/v% or less, and still more preferably 0.01 w/v% or more and 5.0 w/v% or less.

In particular, when one or more types selected from phosphate buffers and borate buffers are used as the buffering agent, the concentration thereof, as the total of the components of the phosphate buffer and borate buffer, is preferably 0.001 w/v% or more and 10 w/v% or less, more preferably 0.01 w/v% or more and 5.0 w/v% or less, and still more preferably 0.1 w/v% or more and 5.0 w/v% or less.

An ophthalmic composition of the present invention may further contain, as necessary, at least one of decongestant components, anti-inflammatory/astringent components, vitamins, cooling agents, isotonizing agents, pH adjusting agents, antioxidants, stabilizing agents, preservatives, mucin secretion promoters, thickeners, and the like, which can generally be used in ophthalmic preparations.

Examples of the decongestant components include epinephrine or salts thereof, ephedrine hydrochloride, tetrahydrozoline hydrochloride, naphazoline or salts thereof, phenylephrine, and methylephedrine hydrochloride.

Examples of the anti-inflammatory/astringent components include epsilon-aminocaproic acid, allantoin, berberine or salts thereof, sodium azulene sulfonate, glycyrrhizic acid or salts thereof, zinc lactate, zinc sulfate, and lysozyme chloride.

Examples of the vitamins include flavin adenine dinucleotide sodium, cyanocobalamin, retinol acetate, retinol palmitate, pyridoxine hydrochloride, panthenol, sodium pantothenate, and calcium pantothenate.

Examples of the cooling agents include menthol and camphor.

Examples of the isotonizing agents include sodium chloride, potassium chloride, magnesium chloride, calcium chloride, aspartic acid or salts thereof, and aminoethylsulfonic acid.

Examples of the pH adjusting agents include citric acid, sulfuric acid, acetic acid, tris hydroxymethylaminomethane, monoethanolamine, sodium hydrogen carbonate, and sodium citrate.

Examples of the antioxidants include tocopheryl acetate, dibutylhydroxytoluene, and sodium bisulfite.

Examples of the stabilizing agents include sodium edetate, glycine, and taurine.

Examples of preservatives include benzalkonium chloride, chlorhexidine gluconate, potassium sorbate, methylparaben, ethylparaben, propylparaben, isopropylparaben, butylparaben, isobutylparaben, polyhexanide hydrochloride, sulfamexazole or salts thereof, sulfisoxazole, and sulfisomidine sodium.

Examples of the mucin secretion promoters include diquafosol sodium and rebamipide.

Examples of the thickeners include poly (meth)acrylic acid, (meth)acrylic acid-acrylic (meth)acrylate copolymer, hydroxyethyl cellulose, hydroxypropyl cellulose, hydroxypropyl methylcellulose, carboxy methylcellulose, polyvinylpyrrolidone, polyvinyl alcohol, and polyethylene glycol.

An ophthalmic composition of the present invention may contain a polysaccharide or a glycoprotein of 0.001 w/v% or more and 10.0 w/v% or less. Examples of the polysaccharide include hyaluronic acid or salts thereof, chondroitin sulfate or salts thereof, alginic acid or a salt thereof, chitosan, pullulan, agar, starch, xanthan gum, gellan gum. Examples of the glycoprotein include proteoglycans. Hyaluronic acid or a salt thereof, chondroitin sulfate or a salt thereof, or a proteoglycan is preferred, and hyaluronic acid or a proteoglycan is more preferred.

In view of irritation to the eye, the pH of an ophthalmic composition of the present invention is preferably pH3.0 or more and 8.0 or less, more preferably 3.8 or more and 7.8 or less, and still more preferably 4.0 or more and 7.6 or less.

In view of irritation to the eye, the osmotic pressure of an ophthalmic composition of the present invention is 200 mOsm or more and 400 mOsm or less, more preferably 225 mOsm or more and 375 mOsm or less, and still more preferably 230 mOsm or more and 340 mOsm or less. The osmotic pressure ratio is preferably 0.7 or more and 1.4 or less, more preferably 0.7 or more and 1.3 or less, and still more preferably 0.8 or more and 1.2 or less.

Herein, the osmotic pressure of a contact lens treatment liquid refers to a value measured according to the 18th Revised Japanese Pharmacopoeia General Test Method 2.47 Osmotic Pressure Measurement Method (Osmolarity Measurement Method), and the osmotic pressure ratio refers to a value obtained by dividing the obtained osmotic pressure value by the osmotic pressure value of 0.9% by mass physiological saline (286 mOsm).

### Examples

Hereinafter, the present invention will be described in more detail based on Examples, but the present invention is not limited thereto. Various measurements in Examples (and Comparative examples) were performed according to the following method.

### Synthesizing copolymer

### <Measurement of weight average molecular weight>

The obtained copolymer was dissolved in ion-exchanged water to a concentration of 0.1 wt %, and the weight average molecular weight of the copolymer was measured by gel-permeation chromatography (GPC).
Column: Shodex(GSM-700)
Mobile phase: Aqueous solution of 0.1 mol/L sulfuric acid
Reference material: Pullulan (PSS-pulkitr1h) (manufactured by Polymer Standards)
Measuring equipment: Differential refractive index RI-8020 (Tosoh Corporation Co., Ltd.)
Method for calculating weight average molecular weight: Molecular Weight Calculation Program (GPC Program for SC-8020)
Flow rate: 1.0 mL per minute
Injection amount: 100 µL
Column oven: 40°C
Measurement time: 30 minutes

### < Example 1-1>

As a GE monomer (a), 3.3 g of 2-guanidinoethyl methacrylate hydrochloride and as a monomer (b), 4.7 g of 2-(methacryloyloxy)ethyl 2-(trimethylammonio)ethyl phosphate (hereinafter, also referred to as MPC) were weighed into a four-neck flask, and 31.0 g of an ion-exchanged water was added thereto, and the mixture was warmed to 70°C and dissolved. Thereafter, 0.06g of 2,2'-azobis(2-methylpropionamidine) dihydrochloride diluted in 1 g of ion-exchanged water was added and polymerized at 70°C for 2 hours. After completion of the reaction, the mixture was dialyzed and purified to obtain 2-guanidinoethyl methacrylate hydrochloride / 2-methacryloyloxyethyl phosphorylcholine copolymer (P1). Polymerization conditions and weight average molecular weight are shown in Table 1. Synthesis of 2-guanidinoethyl methacrylate hydrochloride was performed by the method described in Reference 1 above.

¹H NMR measurements of the copolymer (P1) are shown in the Figure and below. ¹H NMR Measurement result:
C(=O)-O-CH₂-CH₂-O-, P-O-CH₂-CH₂-, C(=O)-O-CH₂-CH₂-NH-: 4.2 to 3.8 ppm, CH₂-N⁺(CH₃)₃, C(=O)-O-CH₂-CH₂-NH-: 3.6 to 3.3 ppm, N⁺(CH₃)₃: 3.1 ppm, C(CH₃)-CH₂-: 2.0 to 1.6 ppm, C(CH₃)-CH₂-: 1.2 to 0.6 ppm

### < Examples 1-2 to 1-6>

The compositions and concentrations of the monomers to be charged were set as shown in Table 1, and the copolymers P2 to P6 were synthesized in the same manner as in Example 1-1. The results of the measurement of the weight average molecular weight are shown in Table 1. Synthesis of 2-guanidinoethyl methacrylamide was performed by the method described in Reference 2 above.

¹H NMR measurements of the copolymers (P2 to P6) are shown below.
¹H NMR Measurement result:
(P2) C(=O)-O-CH₂-CH₂-O-, P-O-CH₂-CH₂-, C(=O)-O-CH₂-CH₂-NH-: 4.2 to 3.8 ppm, CH₂-N⁺(CH₃)₃, C(=O)-O-CH₂-CH₂-NH-: 3.6 to 3.3 ppm, N⁺(CH₃)₃: 3.1 ppm, C(CH₃)-CH₂-: 2.0 to 1.6 ppm, C(CH₃)-CH₂-: 1.2 to 0.6 ppm
(P3) C(=O)-O-CH₂-CH₂-NH-: 4.2 to 3.8 ppm, CH₂-CH-N-: 3.7 ppm, C(=O)-O-CH₂-CH₂-NH-: 3.6 to 3.3 ppm, (C=O)N-CH₂-: 3.2 ppm, N(C=O)-CH₂-: 2.3 ppm, CH₂-CH-N-: 2.0 ppm, C(CH₃)-CH₂-: 2.0 to 1.6 ppm, CH₂-CH₂-CH₂-: 1.7 ppm, C(CH₃)-CH₂-: 1.2 to 0.6 ppm
(P4) C(=O)-O-CH₂-CH₂-O-, P-O-CH₂-CH₂-. 4.2 to 3.8 ppm, CH₂-N⁺(CH₃)₃: 3.6 to 3.3 ppm, C(=O)-NH-CH₂-CH₂-NH-: 3.4 to 3.2 ppm, N⁺(CH₃)₃: 3.1 ppm, C(CH₃)-CH₂-: 2.0 to 1.6 ppm, C(CH₃)-CH₂-: 1.2 to 0.6 ppm
(P5) C(=O)-O-CH₂-CH₂-O-, P-O-CH₂-CH₂-: 4.2 to 3.8 ppm, CH₂-CH-N-: 3.7 ppm, CH₂-N⁺(CH₃)₃, C(=O)-O-CH₂-CH₂-NH-: 3.6 to 3.3 ppm, (C=O)N-CH₂-: 3.2 ppm, N⁺(CH₃)₃: 3.1 ppm, N(C=O)-CH₂-: 2.3 ppm, CH₂-CH-N-: 2.0 ppm, C(CH₃)-CH₂-: 2.0 to 1.6 ppm, CH₂-CH₂-CH₂-: 1.7 ppm, C(CH₃)-CH₂-: 1.2 to 0.6 ppm
(P6) C(=O)-O-CH₂-CH₂-O-, P-O-CH₂-CH₂-, C(=O)-O-CH₂-CH₂-NH-: 4.2 to 3.8 ppm, CH₂-N⁺(CH₃)₃, C(=O)-O-CH₂-CH₂-NH-: 3.6 to 3.3 ppm, N⁺(CH₃)₃: 3.1 ppm, C(CH₃)-CH₂-: 2.0 to 1.6 ppm, C(CH₃)-CH₂-: 1.2 to 0.6 ppm

**[Table 1]**

| | | Examples | | | | | |
|---|---|---|---|---|---|---|---|
| | | 1-1 | 1-2 | 1-3 | 1-4 | 1-5 | 1-6 |
| Molar ratio (%) of monomer (a) | 2-Guanidinoethyl methacrylate | 50 | 20 | 50 | - | 50 | 50 |
| | 2-Guanidinoethyl methacrylamide | - | - | - | 50 | - | - |
| Molar ratio (%) of monomer (b) | MPC | 50 | 80 | - | 50 | 25 | 50 |
| | N-vinylpyrrolidone | - | - | 50 | - | 25 | - |
| Monomer concentration (wt%) | | 20 | 20 | 20 | 20 | 20 | 10 |
| Polymerization solvent | | Ion-exchanged water | | | | | |
| Polymerization initiator | | 2,2'-Azobis(2-methylpropionamidine) dihydrochloride | | | | | |
| Polymerization temperature/time | | 70°C/2 hours | | | | | |
| Weight average molecular weight (kDa) | | 260 | 1320 | 120 | 310 | 220 | 67 |
| Obtained copolymers | | P1 | P2 | P3 | P4 | P5 | P6 |

### < Comparative example 1-1>

Into a four-necked flask, 4.0g of 2-guanidinoethyl methacrylate hydrochloride was weighed. Thereto, 31.0 g of ion-exchanged water was added, and the mixture was warmed to 70°C and dissolved. Thereafter, 0.06g of 2,2'-azobis(2-methylpropionamidine) dihydrochloride diluted in 1.0 g of ion-exchanged water was added and polymerized at 70°C for 2 hours. After completion of the reaction, the mixture was dialyzed and purified to obtain 2-guanidinoethyl methacrylate hydrochloride homopolymer Q1 (weight average molecular weight: 570,000).

### < Comparative example 1-2>

In 26.2g of ion-exchanged water, 12.8 g of MPC was dissolved and placed in a four-necked flask. Nitrogen was blown into the flask for 30 minutes. Thereafter, 0.1g of 2,2'-azobis(2-methylpropionamidine) dihydrochloride diluted in 1.0g of ion-exchanged water was added and polymerized at 70°C for 6 hours. After completion of the reaction, the reaction was dialyzed and purified to obtain a MPC homopolymer Q2 (weight average molecular weight: 330,000).

### < Comparative example 1-3>

N-vinylpyrrolidone homopolymer (PVP K30) (weight average molecular weight: 50,000) (manufactured by Fujifilm Wako Pure Chemical Industries, Ltd.) was used as a comparative polymer Q3.

### 2. Copolymer evaluation

The copolymers of the present invention described above and the copolymers other than those of the present invention were evaluated by the following methods. The test contact lenses used are described below. The solution compositions and evaluation results are shown in Tables 2 to 4.
Lens A: Commercially available Etafilcon A
Lens B: Commercially available Hioxyfilcon A

### < Preparation of physiological saline >

### Physiological saline was prepared with reference to the literature (ISO 18369-3: 2006, Ophthalmic Optics-Contact Lenses Part 3: Measurement Methods.)

That is, 8.3 g of sodium chloride, 5.993 g of sodium hydrogen phosphate dodecahydrate, and 0.528 g of sodium dihydrogen phosphate dihydrate were weighed, dissolved in water to make a total volume of 1000 mL, and filtered to obtain the physiological saline.

### <Preparation of contact lens treatment liquid >

Contact lens treatment liquids of Examples 2-1 to 2-10 and Comparative examples 2-1 to 2-4 were prepared using the ISO physiological saline and the copolymers prepared above by compounding as shown in Table 2 and Table 3.

### <Hydrophilicity Evaluation>

In the Examples and Comparative examples, hydrophilicity evaluation of contact lenses was performed according to the following procedure.
∘ Evaluation assuming the start of contact lens wear
   (1) One test contact lens taken out from a blister pack was immersed in the physiological saline and shaken for 6 hours.
   (2) The test contact lens was taken out and enclosed in a glass vial to which the above contact lens treatment liquid 5 mL was added.
   (3) Sterilization treatment was performed under the conditions of 121°C and 20 minutes.
   (4) The time (BUT) from the taking out of the test contact lens until the water film on the lens surface disrupted was measured with a stopwatch, and the difference from the measured value of a test contact lens treated similarly with the ISO physiological saline was evaluated according to the criteria below.
∘ Evaluation assuming the end of contact lens wear
   (1) One test contact lens taken out from a blister pack was immersed in the physiological saline and shaken for 6 hours.
   (2) The test contact lens was taken out and enclosed in a glass vial to which the above contact lens treatment liquid 5 mL was added.
   (3) Sterilization treatment was performed under the conditions of 121°C and 20 minutes.
   (4) The one test contact lens taken out from the glass vial was immersed in physiological saline and shaken for 6 hours.
   (5) The time (BUT) from the taking out of the test contact lens until the water film on the lens surface disrupted was measured with a stopwatch, and the difference from the measured value of a test contact lens treated similarly with the ISO physiological saline was evaluated according to the criteria below.

The BUT of lens A treated similarly with the ISO physiological saline was 8.4 seconds, and the BUT of lens B was 8.6 seconds.

### Score criteria

Extended for more than 10 seconds: Score 3
Extended for more than 5 seconds and less than 10 seconds: score 2
Extended for more than one second and less than 5 seconds: score 1
Extended for less than one second: score 0

### < Lubricity evaluation>

In the Examples and Comparative examples, lubricity evaluation of contact lenses was performed according to the following procedures.
∘ Evaluation assuming the start of contact lens wear
   (1) One test contact lens taken out from a blister pack was immersed in the physiological saline and shaken for 6 hours.
   (2) The test contact lens was taken out and enclosed in a glass vial to which the above contact lens treatment liquid 5 mL was added.
   (3) Sterilization treatment was performed under the conditions of 121°C and 20 minutes.
   (4) The test contact lens was taken out and rubbed between the index finger and thumb, and the degree of lubricity was evaluated according to the criteria below.
∘ Evaluation assuming the end of contact lens wear
   (1) One test contact lens taken out from a blister pack was immersed in the physiological saline and shaken for 6 hours.
   (2) The test contact lens was taken out and enclosed in a glass vial to which the above contact lens treatment liquid 5 mL was added.
   (3) Sterilization treatment was performed under the conditions of 121°C and 20 minutes.
   (4) The one test contact lens taken out from the glass vial was immersed in physiological saline and shaken for 6 hours.
   (5) The test contact lens was taken out and rubbed between the index finger and thumb, and the degree of lubricity was evaluated according to the criteria below.

### Score criteria

The evaluation score for Polymacon was 2 points, and the evaluation score for Omafilcon A was 8 points, and the scores were calculated within a range of 1 to 10 points. The points were compared with those of test contact lenses treated with ISO physiological saline and evaluated by the criteria below. The evaluation score for lens A treated with ISO physiological saline solution was 3 points, and that of lens B was 5 points.
Increase of 5 points or more: score 3
Increase of 3 to 4 points: score 2
Increase of 1 to 2 points: score 1
Same or less: score 0

### < Durability evaluation >

In the Examples and Comparative examples, a durability evaluation was performed when both the scores for hydrophilicity and lubricity at the start of wearing were 1 or more. The durability evaluation was evaluated on the basis of the evaluations for hydrophilicity and lubricity according to the following criteria.
The scores for both evaluations in assuming the start of wearing and assuming the end of wearing were the same: score 3
The score either one of the evaluations in assuming the start of wearing and assuming the end of wearing was reduced by 1: score 2
The scores for both evaluations in assuming the end of wearing were reduced by 1: score 1
The score for at least one of the evaluations in assuming the end of wearing was reduced by 2 or more: score 0

### < Evaluation of interaction with mucin>

In Examples 3-1 to 3-6 and Comparative examples 3-1 to 3-3, the evaluation of interaction with mucin was performed according to the following procedures. The component compositions and results of the test solutions are shown in Table 4.
(1) Mucin and a polymer were diluted to 10 ppm in the ISO physiological salines, respectively, and DLS measurement was performed using a Zetasizer.
(2) Similar measurement was performed on a mixed solution of the polymer and the mucin.
(3) When the particle size distribution by scattering intensity of the mixed solution had shifted to the higher molecular weight side compared to before mixing, it was considered that there was an interaction with mucin and no remarkable change was observed, it was considered that there was no interaction.

### < Drying resistance >

In Examples 4-1 to 4-6 and Comparative examples 4-1 to 4-4, the drying resistance evaluation was performed according to the following procedure. Lens B was used as the test contact lens. The component composition and results of the test solution are shown in Table 5.
(1) One test contact lens taken out from a blister pack was immersed in the ISO physiological saline and shaken for 6 hours.
(2) Test contact lenses were respectively immersed in glass vials to which 4 mL of corresponding solutions prepared were added as shown in Table 4, and allowed to stand for 3 hours.
(3) Each test contact lens was taken out from the glass vial and allowed to stand at temperatures of 20 to 25°C, 30-35% humidity and under windless conditions. The time to dry and deform was measured and evaluated by the following criteria.

### Score criteria

More than 7 minutes: score 3
More than 6 minutes to less than 7 minutes: score 2
More than 5 minutes to less than 6 minutes: score 1
Less than 5 minutes: score 0

**[Table 2]**

| | | Examples | | | | | | | | | |
|---|---|---|---|---|---|---|---|---|---|---|---|
| | | 2-1 | 2-2 | 2-3 | 2-4 | 2-5 | 2-6 | 2-7 | 2-8 | 2-9 | 2-10 |
| Copolymers used | | P1 | P2 | P3 | P4 | P5 | P6 | P1 | P1 | P1 | P1 |
| Component added (w/v%) | Polymer | 0.1 | 0.1 | 0.1 | 0.1 | 0.1 | 0.1 | 0.02 | 1.0 | 0.1 | 0.1 |
| | Hyaluronic acid | - | - | - | - | - | - | - | - | 0.05 | - |
| | Proteoglycan | - | - | - | - | - | - | - | - | - | 0.05 |
| | ISO physiological saline | Remaining amount in total 100% | | | | | | | | | |
| Test contact lens | | A | A | A | A | A | A | A | A | B | B |
| Appearance/properties | | Colorless and transparent | Colorless and transparent | Colorless and transparent | Colorless and transparent | Colorless and transparent | Colorless and transparent | Colorless and transparent | Colorless and transparent | Colorless and transparent | Colorless and transparent |
| Hydrophilicity (BUT) | Assuming start of wear | 3 | 3 | 3 | 3 | 3 | 3 | 2 | 3 | 3 | 3 |
| | Assuming end of wear | 3 | 2 | 3 | 3 | 3 | 3 | 2 | 3 | 2 | 2 |
| Lubricity (sensation) | Assuming start of wear | 3 | 2 | 3 | 3 | 3 | 3 | 2 | 3 | 2 | 2 |
| | Assuming end of wear | 3 | 2 | 3 | 3 | 3 | 2 | 2 | 3 | 2 | 2 |
| Durability | | 3 | 2 | 3 | 3 | 3 | 2 | 3 | 3 | 2 | 2 |

**[Table 3]**

| | | Comparative examples | | | |
|---|---|---|---|---|---|
| | | 2-1 | 2-2 | 2-3 | 2-4 |
| Copolymers used | | Q1 | Q2 | Q3 | No |
| Component added (w/v%) | Polymer | 0.1 | 0.1 | 0.1 | - |
| | Hyaluronic acid | - | - | - | 0.05 |
| | Proteoglycan | - | - | - | - |
| | ISO physiological saline | Remaining amount in total 100% | | | |
| Test contact lens | | A | A | A | B |
| Appearance/properties | | Colorless and transparent | Colorless and transparent | Deformed | Colorless and transparent |
| Hydrophilicity (BUT) | Assuming start of wear | 0 | 3 | Not performed | 2 |
| | Assuming end of wear | 0 | 1 | Not performed | 0 |
| Lubricity (sensation) | Assuming start of wear | 0 | 1 | Not performed | 1 |
| | Assuming end of wear | 0 | 0 | Not performed | 0 |
| Durability | | - | 0 | - | 0 |

| | | | | | |
|---|---|---|---|---|---|
| * In Comparative example 2-1, scores for the hydrophilicity and lubricity at the start of wearing were 0, and thus the durability evaluation was not performed ** In Comparative example 2-3, the lens was deformed, and thus further evaluations were not performed | | | | | |

**[Table 4]**

| | | Examples | | | | | | Comparative examples | | |
|---|---|---|---|---|---|---|---|---|---|---|
| | | 3-1 | 3-2 | 3-3 | 3-4 | 3-5 | 3-6 | 3-1 | 3-2 | 3-3 |
| Copolymers used | | P1 | P2 | P3 | P4 | P5 | P6 | Q1 | Q2 | Q3 |
| Component added (ppm) | Polymer | 10 | 10 | 10 | 10 | 10 | 10 | 10 | 10 | 10 |
| | Mucin | 10 | 10 | 10 | 10 | 10 | 10 | 10 | 10 | 10 |
| | ISO physiological saline | Remaining amount in total 100% | | | | | | Remaining amount in total 100% | | |
| Interaction | | Yes | Yes | Yes | Yes | Yes | Yes | No | No | No |

**[Table 5]**

| | | Examples | | | | | | Comparative examples | | | |
|---|---|---|---|---|---|---|---|---|---|---|---|
| | | 4-1 | 4-2 | 4-3 | 4-4 | 4-5 | 4-6 | 4-1 | 4-2 | 4-3 | 4-4 |
| Copolymers used | | P1 | P2 | P3 | P4 | P5 | P6 | Q1 | Q2 | Q3 | No |
| Component added (w/v%) | Polymer | 0.1 | 0.1 | 0.1 | 0.1 | 0.1 | 0.1 | 0.1 | 0.1 | 0.1 | - |
| | Mucin | 0.01 | 0.01 | 0.01 | 0.01 | 0.01 | 0.01 | 0.01 | 0.01 | 0.01 | 0.01 |
| | ISO physiological saline | Remaining amount in total 100% | | | | | | Remaining amount in total 100% | | | |
| Drying resistance | | 3 | 3 | 2 | 3 | 3 | 2 | 1 | 1 | 0 | 1 |

### <Evaluation>

From the results of Tables 2 and 3, it was found that the contact lens treatment liquids using copolymer P1 to P6 have improved hydrophilicity and lubricity of the contact lens, and the effects lasted for a long period of time. From the results in Table 4, copolymers P1 to P6 was found to interact with mucin. From the results in Table 5, it was found that copolymers P1 to P6 has a high drying resistance.

This application is entitled to and claims the benefits of Japanese Patent Application No. 2022-204230 filed on December 21, 2022, the disclosure of which including the specification and drawing is incorporated herein by reference in its entirety.

### Industrial Applicability

The copolymer and ophthalmic composition of the present invention can be used as a contact lens treatment liquid to improve the wearing feeling of contact lenses, and can sustain their effectiveness for a long period of time. In addition, when used as eye drops, they can remain on the corneal surface for a longer period of time due to the interaction with mucin, thereby prolonging their moisturizing efficacy.

## Claims

1. A copolymer comprising:
a constituent unit derived from a monomer (a) represented by a formula (1a) below or a salt thereof; and a constituent unit derived from a monomer (b) represented by any one of formulas (1b) and (2b) below: 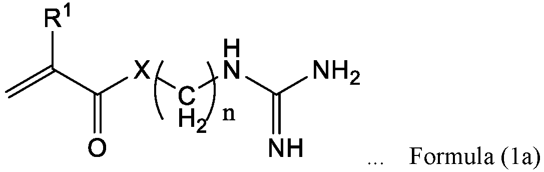 wherein, R¹ is a hydrogen atom or a methyl group, X is O or NR² where R² is H or an alkyl group having 1 to 4 carbon atoms, and n is 1 to 4, 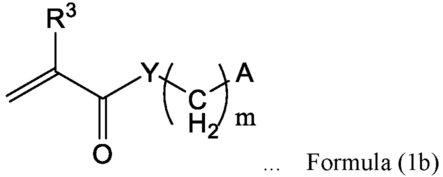 wherein, R³ is a hydrogen atom or a methyl group, Y is O or NR⁴ where R⁴ is H or an alkyl group having 1 to 4 carbon atoms, m is 1 to 6, a carbon chain of the formula (1b) optionally has at least one hydroxyl group, and A is a phosphorylcholine group or a hydroxyl group and optionally is a hydrogen atom only in a case where m is 1 or in a case where the carbon chain has the at least one hydroxyl group, 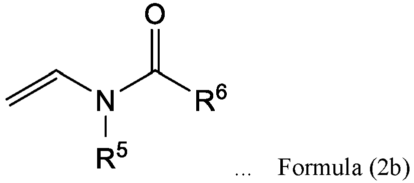 wherein R⁵ is a hydrogen atom or a methyl group, and R⁶ is an alkyl group having 1 to 4 carbon atoms or an alkylene group having 1 to 4 carbon atoms whose terminal is bonded to R⁵ to form a cyclic structure,
wherein
a molar ratio nₐ:n_{b} between the constituent units is 10:90 to 70:30, nₐ being a molar proportion of the constituent unit derived from the monomer (a) or the salt thereof in the copolymer, n_{b} being a molar proportion of the constituent unit derived from the monomer (b) in the copolymer, and
a weight average molecular weight of the copolymer is 10,000 to 2,000,000.

2. The copolymer according to claim 1, wherein
the monomer (b) contains at least one monomer selected from the group consisting of monomers represented by a formula (3b) below, N-vinylpyrrolidone, hydroxyethyl (meth)acrylate, hydroxypropyl (meth)acrylate, hydroxybutyl (meth)acrylate, hydroxyethyl (meth)acrylamide, glycerol mono(meth)acrylate, N,N-dimethyl (meth)acrylamide, N-vinyl acetamide, and N-vinyl-N-methyl acetamide: 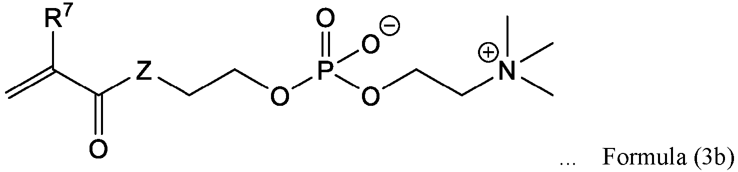 wherein R⁷ is a hydrogen atom or a methyl group, and Z is O or NR⁸ where R⁸ is H or an alkyl group having 1 to 4 carbon atoms.

3. The copolymer according to claim 1 or 2, wherein
the monomer (b) contains at least one of a monomer represented by a formula (3b) and N-vinylpyrrolidone.

4. The copolymer according to any one of claims 1 to 3, wherein
the monomer (b) contains 2-(methacryloyloxy)ethyl 2-(trimethylammonio)ethyl phosphate.

5. The copolymer of any one of claims 1 to 4, wherein
the monomer (a) or the salt thereof contains a monomer represented by a formula (2a) below or a salt thereof: 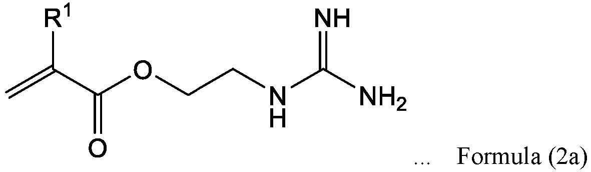 wherein R¹ is a hydrogen atom or a methyl group.

6. An ophthalmic composition comprising:
0.001 w/v% or more and 5.0 w/v% or less of the copolymer (P) according to any one of claims 1 to 5.

7. The ophthalmic composition according to claim 6, further comprising:
0.001 w/v % or more and 10.0 w/v % or less of a polysaccharide or a glycoprotein.
